# EUROPEAN PATENT APPLICATION

(11) **EP 4 821 436 A1**
(43) Date of publication of application: **14.10.2026**
(21) Application number: 25169847.8
(22) Date of filing: 10.04.2025
(51) Int. Cl.: A61B 5/273, A61B 5/259

(54) **METHOD FOR MANUFACTURING AN ARRANGEMENT COMPRISING AN ELECTRODE FOR ATTACHMENT TO THE HUMAN SKIN**

(71) Applicant: Leonhard Lang GmbH, 6020 Innsbruck (AT)
(72) Inventor: LANG, Burrhus, 6020 Innsbruck (AT)
(74) Representative: Torggler & Hofmann Patentanwälte - Innsbruck

(57) **Abstract**

Method for manufacturing an arrangement (1) comprising an electrode (2) for attachment to the human skin and at least one cable (3) connected to the electrode (2), comprising at least the following steps:
- providing an electrode (2), wherein the electrode (2) comprises at least one electrically nonconductive carrier (5) and at least one electrically conductive conductor (6), wherein the conductor (6) is arranged at the carrier (5) and covers the carrier (5) at least partially, the electrode (2) having a contact area (2a) for contacting the conductor (6),
- providing a cable (3), wherein the cable (3) is a flexible flat cable with at least one electrically conductive sheet material (11), wherein the flexible flat cable has at least one insulated surface, and
- mounting the flexible flat cable to the conductor (6) in the contact area (2a) such that an electric contact is established between the conductive sheet material (11) and the conductor (6).

## Description

The present invention concerns a method for producing an arrangement comprising an electrode for attachment to the human skin and at least one cable connected to the electrode, as well as an arrangement comprising an electrode for attachment to the human skin and at least one cable connected to the electrode, preferably manufactured by said method.

Such arrangements are well known from the prior art. Electrodes in such arrangements are usually medical electrodes. They can be used as measuring electrodes to derive electrical signals from the human body or as therapy electrodes to supply electricity to the human body. For this purpose, the electrodes are attached to the skin and generally have an electrically conductive gel or another electrical contact medium on their underside, which is in electrically conductive contact with a conductor of the electrode. A cable is electrically connected to the conductor, via which currents can be derived from the electrode or fed to the electrode.

In the state of the art, a multitude of options for forming a cable for use with electrodes is known. Most commonly, a cable comprises at least one metal strand with a circular cross-section, which is encased in an insulation. Also, multiple strands, sometimes twisted together, are common. Alternatively, cables with strands made from conductive plastics are also known. In some cases, cables with a conductor printed onto insulating material are used. Such conductors can be printed from ink comprising metal or conductive plastic particles. The conductor may be sintered after printing.

Such cables have some disadvantages. Arrangements comprising an electrode for attachment to the human skin and at least one cable connected to the electrode are often disposable, single-use products due to hygiene requirements in the medical field. Meaning, a cable is permanently mounted to the electrode and has to be disposed together with the electrode after use. With commonly used cables comprising strands, this results in a lot of material being disposed of, resulting in low sustainability and high costs for disposal.

Printed cables usually use less material. However, they do not fulfil the requirements regarding their electrical properties, especially the requirements for low internal resistance, in particular in the case of measuring electrodes. This is due to the resulting material properties of a printed conductor as well as the limited cross-section achievable with printed cables. The same applies for sputtered cables or the like.

The objective technical problem to be solved by the invention is to provide an improved method as well as an improved arrangement, in which the disadvantages of the prior art are at least partially avoided.

This problem is solved by a method according to claim 1 and an arrangement according to claim 5.

According to the invention, at least the following steps are provided with a method for manufacturing an arrangement comprising an electrode for attachment to the human skin and at least one cable connected to the electrode as mentioned above:
- providing an electrode, wherein the electrode comprises at least one electrically non-conductive carrier and at least one electrically conductive conductor, wherein the conductor is arranged at the carrier and covers the carrier at least partially, the electrode having a contact area for contacting the conductor,
- providing a cable, wherein the cable is a flexible flat cable with at least one electrically conductive sheet material, wherein the flexible flat cable has at least one insulated surface, and
- mounting the flexible flat cable to the conductor in the contact area such that an electric contact is established between the conductive sheet material and the conductor.

Using a flexible flat cable in connection with an electrode results in numerous advantages over commonly used cables.

With respect to cables with strands, the amount of materials needed to produce a flexible flat cable with a similar internal resistance can be reduced significantly. Thus, sustainability of single-use arrangements with flexible flat cables is much higher when compared to cables used in the prior art. Also, costs for disposal can be reduced. Additionally, the cost for manufacturing as well as transporting such arrangements are lower due to reduced material expenses and a reduced weight.

With respect to printed cables, a flexible flat cable fulfils the requirements regarding their electrical properties, especially internal resistance, while still using less material, as discussed above.

Furthermore, a desired cross-section of the cable can be easily achieved by selecting an appropriately thick sheet material and adjusting the width of the sheet material. Also, a flexible flat cable can be easily and directly bonded to the conductor of the electrode, foregoing the need for additional connecting elements or complex process for connecting the cable, such as riveting.

Although a person skilled in the art is well aware of the meaning of the term "flexible flat cable", it shall briefly be described how a flexible flat cable differs from cables in the prior art.

With respect to common cables with strands, the cables as well as their cable conductors (the strands) are of a circular cross section. Flexible flat cables are, as by their name, flat. However, not only the flexible flat cable itself is flat but also the cable conductor of the flexible flat cable (the sheet material). This distinguishes flexible flat cable from ribbon cables, with flexible flat cables being thinner than ribbon cables. Thus, a sheet material is an element whose thickness is small compared to its other dimensions.

Printed (or sputtered) cables may also be formed in a flat manner as described above. However, a printed cable conductor of a cable is not comparable to a sheet material which serves as cable conductor of flexible flat cables.

A sheet material differs from a printed cable conductor, inter alia, thereby that the sheet material is structurally integral by itself. Meaning, no base material or the like is needed to support the sheet material. In other words, the sheet material is self-supporting.

In contrast, a printed (or sputtered) cable conductor necessarily needs some form of base material onto which the cable conductor is printed. Also, printed cable conductors usually are less flexible than a sheet material due to their different material properties.

Regarding an arrangement according to the invention it is provided that the arrangement comprises an electrode for attachment to the human skin and at least one cable connected to the electrode, wherein the electrode comprises at least one electrically non-conductive carrier and at least one electrically conductive conductor, wherein the conductor is arranged at the carrier and covers the carrier at least partially, wherein the cable is, preferably directly, electrically connected to the conductor, characterized in that the cable is a flexible flat cable with at least one electrically conductive sheet material.

Further advantageous embodiments of the invention are defined in the dependent claims.

It may be provided that the cable is mounted to the conductor in the contact area by means of material bonding, in particular by adhesive bonding and/or soldering. This allows for an easy and cost-effective connection of the cable to the electrode.

A contact area may be provided on a front side of the conductor facing the skin of a patient, or on a back side of the conductor facing away from the skin toward the carrier. The contact area may also be provided on both the front and the back side of the carrier.

In the latter case, an opening may be provided in the carrier and the contact area may be in said opening.

In a preferred embodiment, it may be provided that the flexible flat cable comprises a flat, flexible and electrically non-conductive base layer, wherein the sheet material is arranged on the base layer, wherein the sheet material is, preferably directly, connected to the conductor, and/or the base layer is, preferably directly, connected to the carrier and/or the conductor.

The sheet material may additionally be connected to the carrier.

These features, together or in isolation, allow to provide the most suitable configuration for connecting the cable to the electrode depending on the specific electrode and its use case.

It can be provided that the sheet material is connected to the conductor by means of an electrically conductive adhesive, and/or the base layer is connected to carrier and/or the conductor by means of an adhesive.

The sheet material may additionally be connected to the carrier by means of an adhesive or an electrically conductive adhesive.

This allows for easy and cost-effective attachment of the cable to the conductor of the electrode.

The sheet material may be flexible and flat.

In view of an arrangement according to the invention it may be provided that the flexible flat cable comprises a flat, flexible and electrically non-conductive base layer, wherein the sheet material is arranged on the base layer.

This is a simple and uncomplicated embodiment of a flexible flat cable.

In a preferred embodiment it may be provided that the base layer is made of plastic and/or the sheet material comprises at least one metal, preferably is made of at least one metal.

The use of plastic as base layer is an easy way of achieving the requirements of the base layer in view of flexibility and electrical insulation in a cost-effective manner. The use of metal with the sheet material helps to reduce the internal resistance of the sheet material and thus to fulfil the requirements regarding the electrical properties of a cable.

It may be advantageously provided that the sheet material is a sheet metal.

Further, the sheet material may
- be formed by a rolling process, and/or
- have a density of at least 99 %, preferably a density of 100 %, and/or
- have a resistivity of between 0.01 Ωmm²/m and 2 Ωmm²/m.

In a preferred embodiment, the resistivity of the sheet material may be between 0.01 Ωmm²/m and 0.72 Ωmm²/m or between 0.01 Ωmm²/m and 0.15 Ωmm²/m.

Especially the resistivity of the sheet material may be 0.0265 Ωmm²/m (aluminium sheet material), between 0.1 Ωmm²/m and 0.2 Ωmm²/m (steel sheet material), 0.01721 Ωmm²/m or between 0.0169 Ωmm²/m and 0.0175 Ωmm²/m (copper sheet material) or 0.01587 Ωmm²/m (sliver sheet material).

The values given above refer to the resistivity at 20° C.

The sheet material may comprise and/or be made of aluminium, steel, copper and/or silver.

Also, the sheet material may have
- a substantially rectangular cross-section, and/or
- a thickness between 8 µm and 500 µm, and/or
- a width between 0.1 mm and 20 mm.

Preferably, the sheet material may have a thickness between 8 µm and 200 µm, or between 18 µm or 20 µm and 100 µm.

Preferably, the sheet material may have a width between 0.1 mm and 10 mm, or between 1 mm and 10 mm.

These features, together or isolated, effect a reduction of the internal resistance of the sheet material and thus contribute to fulfilling the requirements regarding the electrical properties of the cable.

Preferably it may be provided that the cable comprises a flat, flexible and electrically non-conductive top layer, wherein the sheet material is at least partially arranged in between the base layer and the top layer.

Thus, it is possible to fully insulate the conductive sheet material from the environment to prevent defects while using the arrangement and/or to prevent injuries of a patient when using the arrangement.

In a preferred embodiment, the cable may comprise a stabilizing element, wherein the stabilizing element defines a minimum admissible bending radius of the cable.

Thus, snapping of the cable can be prevented.

It may be provided that the stabilizing element consists of at least one fibre arranged at or within the cable.

This is an easy and cost-effective way to realize a stabilizing element.

It may be provided that the sheet material is**,** preferably directly, connected to the conductor, and/or the base layer is, preferably directly, connected to the carrier and/or the conductor.

By directly connecting the sheet material to the conductor, additional connecting elements or the like can be omitted.

Further, it may be provided that the mechanical attachment of the cable is provided at a different location and/or at the same location as the electrical attachment.

In particular, the cable can be electrically connected to the conductor and mechanically connected to the electrode conductor and/or the carrier.

The means for electrically connecting the cable and the means for mechanically connecting the cable can be the same or different means.

The features mentioned above allow, together or distinct from each other, to provide the most suitable configuration for connecting the cable to the electrode depending on the specific electrode and its use case.

Preferably it may be provided that the sheet material is connected to the conductor by means of an electrically conductive adhesive, and/or the base layer is connected to carrier and/or the conductor by means of an adhesive.

The use of a conductive adhesive provides a simple and cost-effective way to electrically connect the cable to the conductor.

It may also be provided that the cable is connected to the conductor at a back side of the conductor which faces toward the carrier.

Thus, a homogenous current distribution can be achieved at the opposite front side of the conductor, which is free from any connecting elements or the cable itself. Additionally, the handling of an arrangement may be improved.

In a preferred embodiment, the cable may be electrically connected to the conductor in an opening provided in the carrier.

Such an opening allows for easy access to the back side of the conductor.

Preferably, such an opening may be manufactured by removing the carrier in at least one defined area by means of a machining process, preferably milling and/or laser ablation, so that an opening for contacting the conductor through the carrier is provided in the carrier.

A machining process may refer to a traditional machining process, where a material is removed by the removal of chips via at least one (defined or undefined) cutting edge, e.g**.** milling. However, a machining process may also refer to the removal of material via a laser, e.g**.** laser ablation.

Due to the carrier being removed from the conductor, it is possible to connect the cable to the back side of the conductor in an easy manner and without the need for an additional connecting element. This goes along with a variety of advantages.

First, a homogenous current distribution can be achieved at the front side of the conductor. Second, the process for attaching the cable to the back side of the conductor does not require any complex steps, such as fanning the strands of a cable, riveting or insulation steps.

Furthermore, the costs for producing an electrode can be reduced as there is no additional material (like a connecting element) and fewer process steps needed. Therein, even small savings regarding material and/or process steps can result in a large cost reduction, as medical electrodes are mass-produced products that are manufactured in very large quantities.

Also, new electrode geometries are possible, since the front side of the conductor is not needed for connecting the cable to the conductor.

In a further embodiment it may be provided that the electrode comprises an electrically conductive contact medium arranged on the conductor, which covers the conductor at least partially.

The contact medium is used to make electrical contact with the skin. The contact medium can be in the form of a conductive gel or a conductive adhesive, for example.

In an advantageous embodiment the electrode may comprise a cover element arranged on the contact medium, which covers the contact medium at least partially.

The cover element can protect the contact medium from damage and soiling.

The covering element can be designed as an abhesive film, for example. This allows for an easy removal of the covering element before using the electrode.

In a further embodiment, it may be provided that an impedance layer is arranged at least partly on the conductor.

The impedance layer can also be electrically conductive. By partially removing the impedance layer from the conductor, the current output from the electrode to the human skin can be altered.

It may also be provided that a plaster layer extending beyond the carrier is arranged on the carrier. The plaster layer is arranged on a side of the carrier on which no conductor is arranged.

The plaster layer can be used to achieve a more secure hold of the electrode on the skin.

Further details and advantages of preferred embodiments of the invention are explained in more detail below with reference to the drawings. Therein shows
- Fig. 1: a first step of a method for manufacturing an arrangement,
- Fig. 2A-F: different further steps of a method for manufacturing the arrangement,
- Fig. 3-8: further steps of a method for manufacturing the arrangement,
- Fig. 9A-B: schematic views of an electrode,
- Fig. 10A-E: schematic views of different embodiments of a cable,
- Fig. 11A-B: schematic views of the arrangement,
- Fig. 12A-D: schematic views of a further embodiment of an electrode,
- Fig. 13A-B: schematic views of a further embodiment of an arrangement,
- Fig. 14: schematic view of a further embodiment of an arrangement,
- Fig. 15A-B: schematic views of two different embodiments of an electrode,
- Fig. 16A: a cross-sectional view of an embodiment of a flat cable with a printed conductor according to the prior art, and
- Fig. 16B: a cross-sectional view of an embodiment of a flexible flat cable.

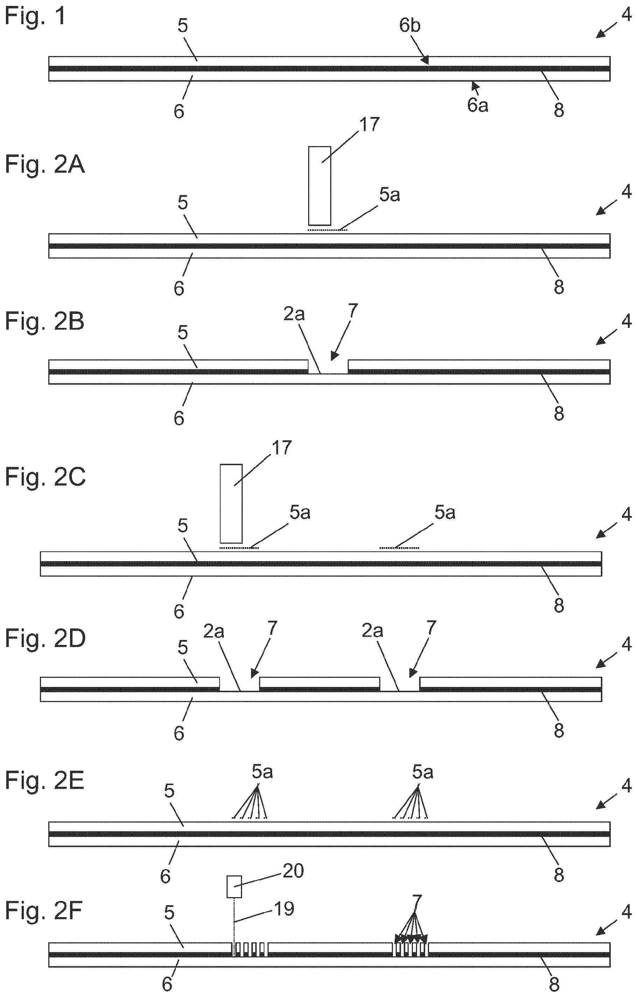

From Figs. 1 to 8, a preferred embodiment of a method for manufacturing of an embodiment of an arrangement 1 can be seen. The Figs. 1 to 8 thereto provide a schematic view of a cross-section of an arrangement 1 at different points during a manufacturing process.

In a first step, a conducting element 4 is provided. The conducting element 4 comprises an electrically non-conductive carrier 5 and an electrically conductive conductor 6. The carrier 5 and the conductor 6 are bonded together by a bonding agent, the bonding agent forming a bonding agent layer 8.

In the present embodiment, the conducting element 4 thus is a conducting composite foil.

The carrier 5 may be made of a plastic, for example from such as polyester or polysterol. The conductor 6 may comprise at least one metal and preferably is made from at least one metal. However, the conductor may also be made from a conductive plastic foil.

The conductor 6 may at least partly be covered by a suitable redox-pair for depolarizing the electrode 2.

The conductor 6 comprises a front side 6a, facing toward the skin of a patient, and a back side 6b, facing toward the carrier 5 and thus away from the skin of a patient.

The front side 6a and back side 6b are thus substantially opposite as well as parallel to each other.

Figs. 2A and 2B, 2C and 2D as well as 2E and 2F disclose different embodiments of a further step for manufacturing an arrangement 1.

It can be seen that a defined area 5a of the carrier 5 is set (dotted lines), wherein the carrier 5 is subsequently removed in the defined areas 5a. This is achieved via machining, in particular via milling with a milling cutter 17. The bonding agent layer 8 is also removed in the defined areas 5a during this process, exposing the conductor 6 on the back side 6b in said defined areas 5a.

Via the machined openings 7, the conductor 6 can be accessed on the back side 6b to connect a cable 3 to the conductor 6. A contact area 2a is thus provided at the back side of the conductor 6 in the opening 7.

In Figs. 2A and 2B, only one opening 7 is produced, whereas two openings 7 are provided in Figs. 2C and 2D. In Fig. 2E and 2F, multiple slit-like openings 7 are produced. Such slit-like openings 7 may be produce via machining with a laser beam 19 produced by a laser 20.

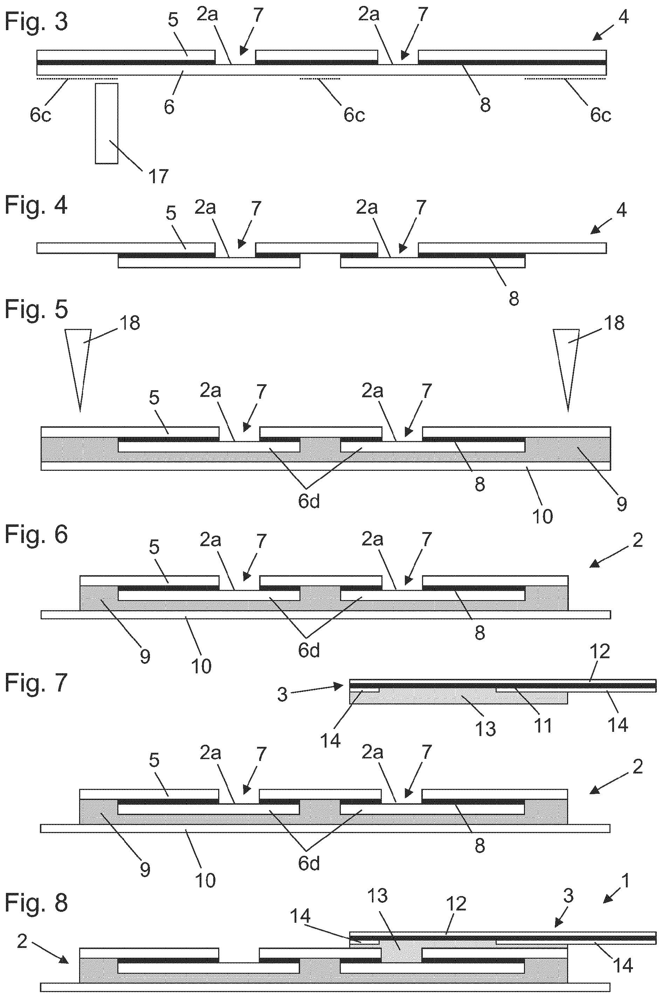

Fig. 3 shows a further step for manufacturing of an arrangement 1, on basis of the steps shown in Figs. 2C and 2D. On the conductor 6, defined areas 6c are set and subsequently the conductor 6 is removed in said defined areas 6c. This is achieved via machining, in particular via milling with a milling cutter 17. The milling cutter 17 may be the same or a different milling cutter 17 than in the previous steps.

Again, the bonding agent layer 8 is removed in the defined areas 6c during this process. However, the bonding agent layer 8 may also remain in the defined areas 6c.

Thereby, two conductor sections 6d are produced, the conductor sections 6d being separate from each other. In particular, the conductor sections 6d are electrically isolated from each other. This can be seen from Fig. 4 as well as Figs. 9A, 9B, 12A, 12B or 13A for example.

In a further step, a contact medium 9 is applied onto the conductor sections 6d as well as the space around the conductor sections 6d.

In a further step, a cover element 10 is applied to the contact medium 9.

Fig. 5 shows the state of the arrangement 1 after the aforementioned steps. In Fig. 5, punching knives 18 can be seen. By means of the punching knives 18, the carrier 5, and, if applicable, the conductor 6, and the contact medium 9 can be cut to define the shape of an electrode 2.

Alternatively or additionally to the punching knives 18, other types of knives may be provided, for example kiss cutting knives.

The cover element 10 is not cut in the figure shown. However, the cover element 10 may also be cut in the same or a different shape by the same or different punching knives 18.

After cutting, an electrode 2 is present, as seen in Fig. 6.

In a further step, a cable 3 is provided. In the present embodiment, the cable 3 is a flexible flat cable with an electrically conductive sheet material 11. The sheet material 11 is arranged on a flat, flexible and electrically non-conductive base layer 14.

Further, a flat, flexible and electrically non-conductive top layer 12 is provided, wherein the sheet material 11 is electrically insulated from the environment by means of the top layer 12 and the base layer 14.

The sheet material 11 is also flat and flexible. In the present embodiment, the sheet material 11 is a sheet metal, for example produced by a rolling process.

An opening 14a is provided in the base layer 14, wherein the sheet material 11 is accessible through the opening 14a.

In the present embodiment, an adhesive 13 is provided with cable 3. The adhesive 13 is provided in the opening 14a on the sheet material 11 as well as on parts of the base layer 14. The adhesive 13 an electrically conductive adhesive 13.

The adhesive 13 may additionally or alternatively be provided on the electrode 2.

In a further step, the cable 13 is connected to the conductor 6 in the contact area 2a in the opening 7 by means of the electrically conductive adhesive 13. Thus, an electric contact is established between the conductor 6 and the sheet material 11 by means of the electrically conductive adhesive 13.

Further, a mechanical connection between the cable 3 and the electrode 2 is achieved via the adhesive 13.

In this embodiment, the sheet material 11 is connected to the connector 6 as well as the carrier 5 via the adhesive 13. The base layer 14 is connected to the carrier 5.

It should be noted that the above-mentioned steps may also be performed in a different order and/or at least some of the steps may be performed parallel to each other.

Also, not all steps are absolutely necessary. It may be provided that only some of the aforementioned steps are performed to manufacture an arrangement.

Especially, it may be provided that the electrode 2 is not manufactured from scratch but rather directly provided. The electrode provided may therein also be manufactured with a different process to the one described above.

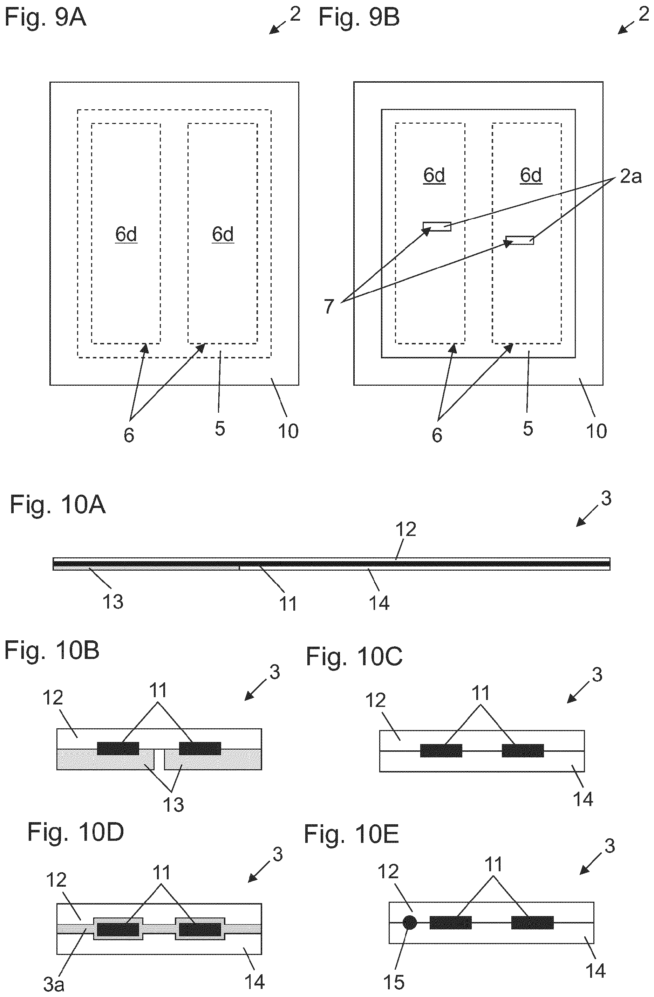

Fig. 9A shows a schematic view on a patient side of an electrode 2, facing toward the patient when in use. Fig. 9B shows a schematic view on an opposite side of an electrode 2, facing away from the patient when in use.

The dashed lines indicate that the corresponding element is hidden underneath another element, as is common in technical drawings.

It can be seen that the electrode 2 comprises a conductor 6 with two conductor sections 6d. The conductor sections 6d are separate from each other and in particular electrically insulated from each other.

The conductor 6 is arrange on a carrier 5. A contact medium 9 is applied on the conductor 6 and the carrier 5, with a cover element 10 arranged thereon. The cover element 10 extends beyond the rest of the electrode 2, which allows for an easier removal of the cover element 10.

It can be seen that the electrode 2 comprises two openings 7 in the carrier 5, wherein the openings 7 are displaced in a longitudinal direction as well as a transversal direction of the electrode 2.

Fig. 10A shows a length-wise cross-section of cable 3. Again, the cable 3 is a flexible flat cable. In contrast to the cable 3 shown in Figs. 7 and 8, the opening 14a in the base layer 14 extends to an end of the cable 3.

In this embodiment, an electrically conductive adhesive 13 can be provided in the opening 14a.

Fig. 10B shows a transversal cross section of the cable 3 in an area of the opening 14a and Fig. 10C in any other area.

It can be seen that two patches of adhesive 13 are provided, each patch being arranged on one of the sheet materials 11. The patches of adhesive 13 are separated from each other to avoid a short circuit.

However, if an electrically conductive adhesive is provided, which is only conductive in a direction orthogonal to the sheet materials 11, only one patch of adhesive covering the whole cross-section of the cable 3 may be provided.

It can be seen that two flat sheet materials 11 are provided between the top layer 12 and the base layer 14. The sheet materials 11 each have a rectangular cross-section.

In Fig. 10D a bonding agent 3a which bonds the parts of cable 3 together is highlighted.

Fig. 10E shows a further embodiment of a cable 3. The cable 3 additionally comprises a stabilizing element 15 in form of at least one fibre arranged at or within the cable 3. The fibre may be made of nylon or the like.

Such a stabilizing element 15 defines a minimum admissible bending radius of the cable 3, thus preventing snapping of the cable 3, in particular the sheet material 11, due to bending of the cable 3.

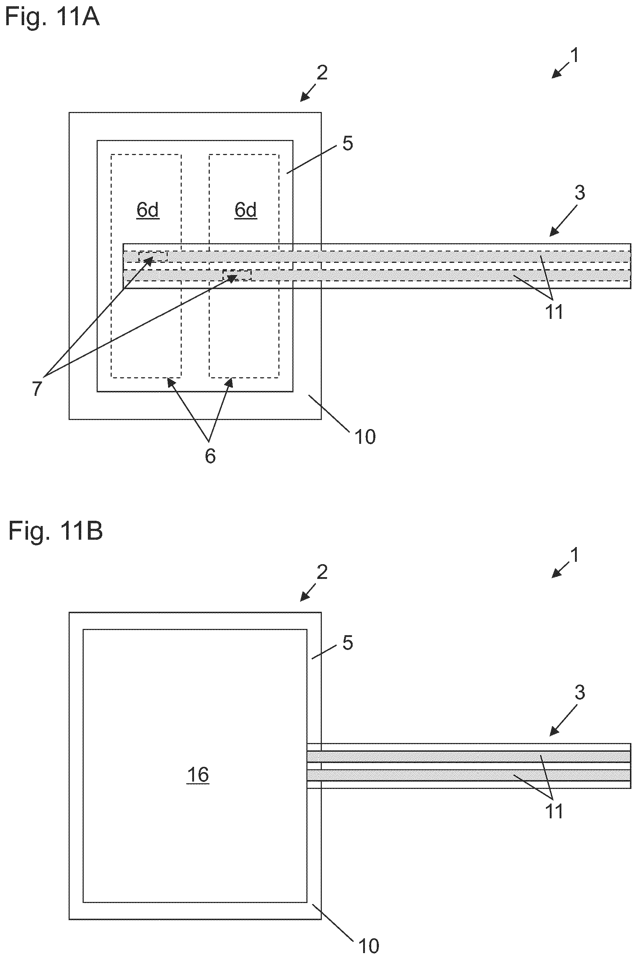

Figs. 11A and 11B show a schematic view on a side of an arrangement 1 facing away from the patient when in use.

It can be seen that the two sheet materials 11 of the cable 3 line up with the two openings 7 in the carrier 5 of the electrode 6. Each of the sheet materials 11 is thus electrically connected with one of the two conductor sections 6.

In Fig. 11B an additional plaster layer 16 is arranged on the carrier 5. This plaster layer 16 can be, exclusively or additionally, be use to fix the arrangement 1 on the skin of a patient. Further, the plaster layer 16 additionally fixes the cable 3 to the electrode 2, increasing the stability of the connection between electrode 2 and cable 3.

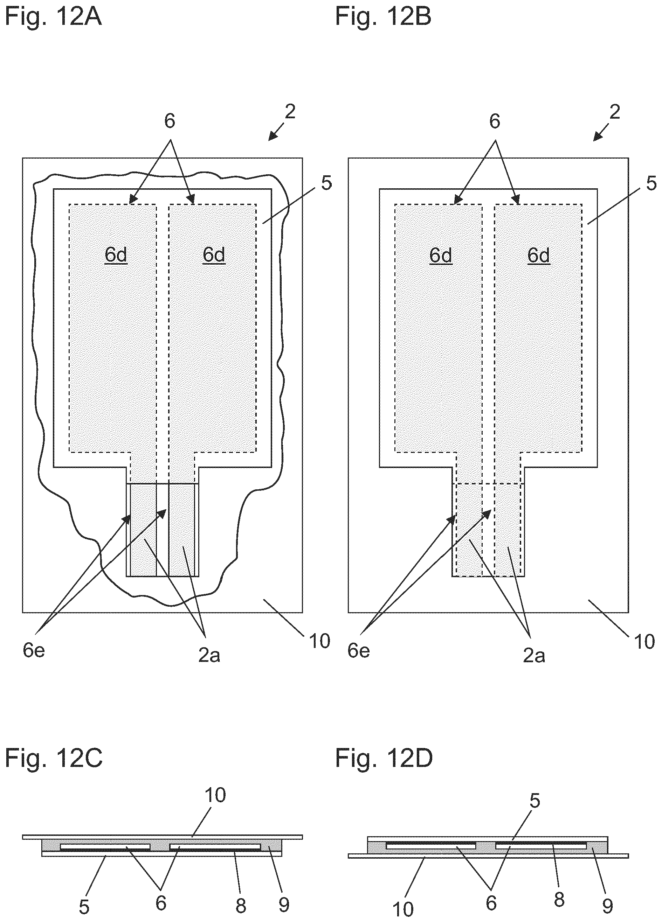

Fig. 12A shows a schematic view on a patient side of a further embodiment of an electrode 2, facing toward the patient when in use. Fig. 12B shows a schematic view on an opposite side of the further embodiment of the electrode 2, facing away from the patient when in use. Figs. 12C and 12D show cross sections of the further embodiment of the electrode 2 in the respective orientation.

It can be seen that no opening in the carrier 5 is provided in this embodiment. Rather, extensions 6e of the conductor sections 6d are provided on the carrier 5, the extension 6e extending beyond the contact medium 9. The contact areas 2a are provided on the extensions 6e.

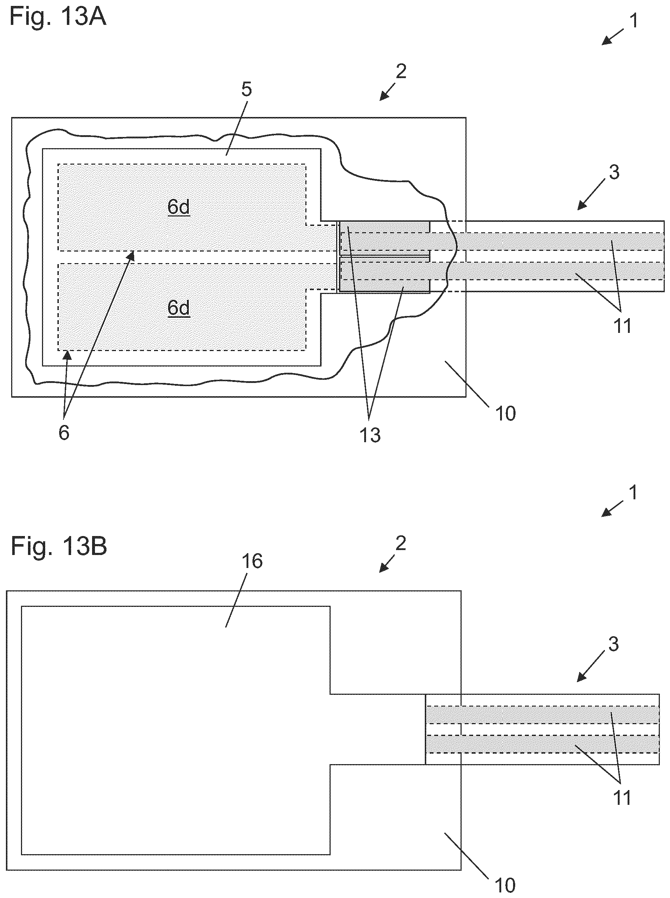

Fig. 13A shows a schematic view on a patient side of a further embodiment of an arrangement 1, facing toward the patient when in use. Fig. 13B shows a schematic view on an opposite side of the further embodiment of the arrangement 1, facing away from the patient when in use.

It can be seen that a cable 3 in form of a flexible flat cable is provided. The cable 3 is attached to the electrode 2 in the area of the contact areas 2a by means of an electrically conductive adhesive. Thus, each of the sheet materials 11 is electrically connected to one of the conductor sections 6d.

Additionally, the top layer 12 of the cable 3 electrically insulates the extensions 6e of the conductor 6 against the environment.

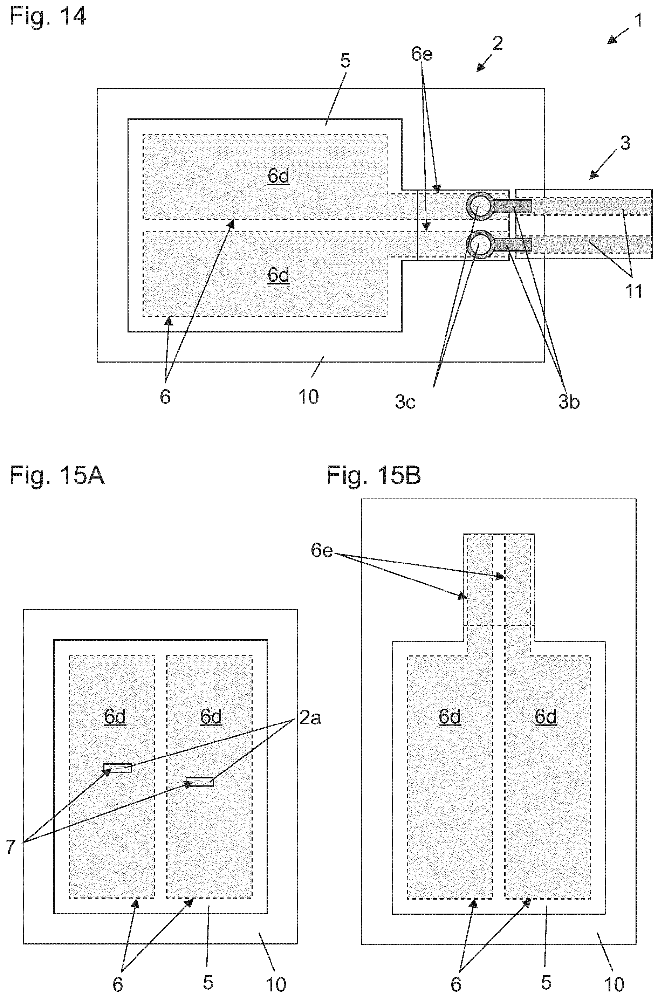

Fig. 14 shows a schematic view of a further embodiment of an arrangement 1. This embodiment differs from the embodiment shown in Figs. 13A and 13B in that no adhesive 13 is used to connect the cable 3 to the electrode 2. Rather, the sheet materials 11 are each provide with a cable lug 3b. The cable lugs 3b are then each riveted to one of the extensions 6e via a rivet 3c.

Figs. 15A and 15B show schematic views of two different embodiments of an electrode 2. It can be seen that the embodiment according to Fig. 15, which utilizes openings 7 in the carrier 5, uses less material in view of the carrier 5, the conductor 6 and the cover element 10. Additionally, said electrode 2 is also smaller and thus easier to handle.

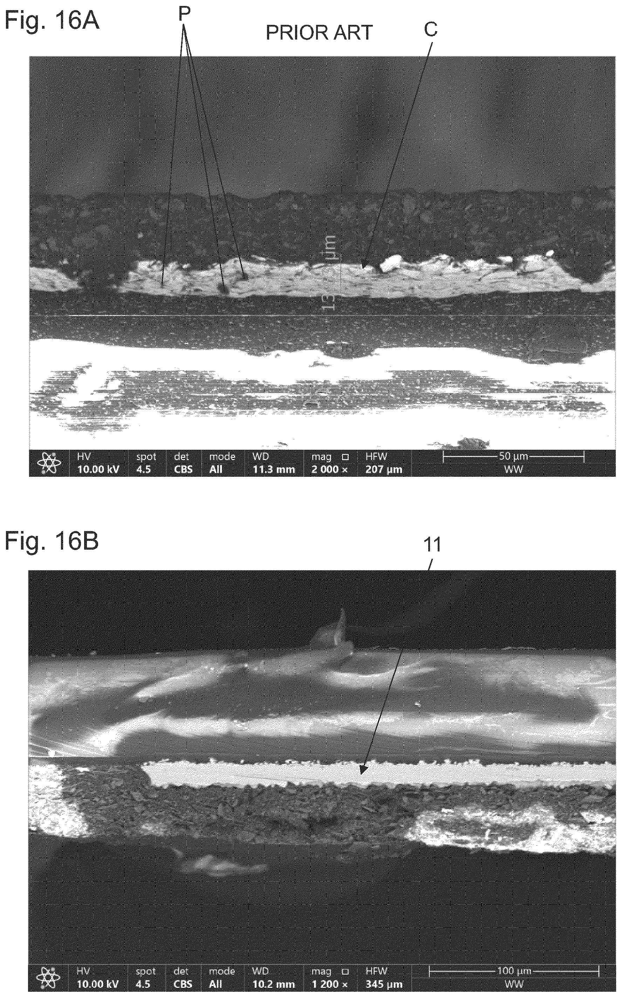

Fig. 16A shows a cross-sectional view of an embodiment of a flat cable 3 with a printed conductor C, and Fig. 14B a cross-sectional view of an embodiment of a flexible flat cable 3.

From these figures the difference between a flat, printed cable 3, as known from the prior art to a flexible flat cable 3 as used with the invention can be clearly seen.

The printed conductor C shows a multitude of pores P enclosed therein, which are a result of the printing process. As described above, such pores P have a significant negative impact on the electrical properties of the conductor C.

As can be seen from Fig. 16B, the sheet material 11 of the flexible flat cable 3 shows a clean metallic cross-section without pores P. This can be achived by using a sheet metal for example.

### List of reference signs:

1 Arrangement
2 Electrode
   2a contact area
3 Cable
   3a bonding agent
   3b cable lug
   3c rivet
4 Conducting element
5 Carrier
   5a defined area
6 Conductor
   6a front side
   6b back side
   6c defined area
   6d conductor section
   6e extension
7 Opening
8 Bonding agent layer
9 Contact medium
10 Cover element
11 Sheet material
12 Top layer
13 Adhesive
14 Base layer
   14a opening
15 Stabilizing element
16 Plaster layer
17 Milling cutter
18 Punching knife
19 Laser beam
20 Laser

## Claims

1. Method for manufacturing an arrangement (1) comprising an electrode (2) for attachment to the human skin and at least one cable (3) connected to the electrode (2), comprising at least the following steps:
- providing an electrode (2), wherein the electrode (2) comprises at least one electrically non-conductive carrier (5) and at least one electrically conductive conductor (6), wherein the conductor (6) is arranged at the carrier (5) and covers the carrier (5) at least partially, the electrode (2) having a contact area (2a) for contacting the conductor (6),
- providing a cable (3), wherein the cable (3) is a flexible flat cable with at least one electrically conductive sheet material (11), wherein the flexible flat cable has at least one insulated surface, and
- mounting the flexible flat cable to the conductor (6) in the contact area (2a) such that an electric contact is established between the conductive sheet material (11) and the conductor (6).

2. Method according to claim 1, wherein the cable (3) is mounted to the conductor (6) in the contact area (2a) by means of material bonding, in particular by adhesive bonding and/or soldering.

3. Method according to claims 1 or 2, wherein the cable (3) comprises a flat, flexible and electrically non-conductive base layer (14), wherein the sheet material (11) is arranged on the base layer (14), wherein the sheet material (11) is, preferably directly, connected to the conductor (6), and/or the base layer (12) is, preferably directly, connected to the carrier (5) and/or the conductor (6).

4. Method according to claim 3, wherein the sheet material (11) is connected to the conductor (6) by means of an electrically conductive adhesive (13), and/or the base layer is connected to carrier (5) and/or the conductor (6) by means of an adhesive (13).

5. Arrangement (1) comprising an electrode (2) for attachment to the human skin and at least one cable (3) connected to the electrode (2), preferably manufactured by a method according to one of claims 1 to 4, wherein the electrode (2) comprises at least one electrically non-conductive carrier (5) and at least one electrically conductive conductor (6), wherein the conductor (6) is arranged at the carrier (5) and covers the carrier (5) at least partially, wherein the cable (3) is, preferably directly, electrically connected to the conductor (6), **characterized in that** the cable (3) is a flexible flat cable with at least one electrically conductive sheet material (11).

6. Arrangement (1) according to claim 5, wherein the cable (3) comprises a flat, flexible and electrically non-conductive base layer (14), wherein the sheet material (11) is arranged on the base layer (14).

7. Arrangement (1) according to claim 6, wherein the base layer (14) is made of plastic and/or the sheet material (11) comprises at least one metal, preferably is made of at least one metal.

8. Arrangement (1) according to claim 7, wherein the sheet material (11)
- is formed by a rolling process, and/or
- has a density of at least 99 %, preferably a density of 100 %, and/or
- has a resistivity between 0.01 Ωmm²/m and 2 Ωmm²/m.

9. Arrangement (1) according to one of claims 7 or 8, wherein the sheet material (11) has
- a substantially rectangular cross-section, and/or
- a thickness between 8 µm and 500 µm, and/or
- a width between 0.1 mm and 20 mm.

10. Arrangement (1) according to one of claims 6 to 9, wherein the cable (3) comprises a flat, flexible and electrically non-conductive top layer (12), wherein the sheet material (11) is at least partially arranged in between the base layer (14) and the top layer (14).

11. Arrangement (1) according to one of claims 5 to 10, wherein the cable (3) comprises a stabilizing element (15), wherein the stabilizing element (15) defines a minimum admissible bending radius of the cable (3), preferably wherein the stabilizing element (15) consists of at least one fibre arranged at or within the cable (3).

12. Arrangement (1) according to one of claims 6 to 11, wherein the sheet material (11) is, preferably directly, connected to the conductor (6), and/or the base layer (14) is, preferably directly, connected to the carrier (5) and/or the conductor (6).

13. Arrangement (1) according to claim 12, wherein the sheet material (11) is connected to the conductor (6) by means of an electrically conductive adhesive, and/or the base layer (12) is connected to carrier (5) and/or the conductor (6) by means of an adhesive.

14. Arrangement (1) according to one of claims 5 to 13, wherein the electrode (2) comprises
- an electrically conductive contact medium (9) arranged on the conductor, which covers the conductor at least partially, and/or

15. Arrangement according to one of claims 5 to 14, wherein the electrode comprises a cover element arranged on the contact medium, which covers the contact medium at least partially.
